(19) Europäisches Patentamt
European Patent Office
Office européen des brevets

(11) Veröffentlichungsnummer: **0 421 266 A1**

# (12) EUROPÄISCHE PATENTANMELDUNG

(21) Anmeldenummer: **90118529.8**

(22) Anmeldetag: **27.09.90**

(51) Int. Cl.5: **A01N 43/88**, A01N 43/66, A01N 43/54, C07D 403/04, //(A01N43/88,43:66,43:54)

(30) Priorität: **06.10.89 US 418222**

(43) Veröffentlichungstag der Anmeldung: **10.04.91 Patentblatt 91/15**

(84) Benannte Vertragsstaaten: **BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Zipplies, Matthias, Dr.**
**Kastanienweg 1**
**W-6945 Hirschberg(DE)**
Erfinder: **Sauter, Hubert, Dr.**
**Neckarpromenade 20**
**W-6800 Mannheim 1(DE)**
Erfinder: **Tuerk, Wolfgang, Dr.**
**Wilhelm-Busch-Strasse 8**
**W-6703 Limburgerhof(DE)**
Erfinder: **Moore, Barbara Auxier**
**Route 4, Box 162**
**Pittsboro, N.C. 27312(US)**
Erfinder: **Carlson, Dale R., Dr.**
**107 King Street**
**Hillsborough, N.C. 27278(US)**
Erfinder: **Wuerzer, Bruno, Dr.**
**Ruedigerstrasse 13**
**W-6701 Otterstadt(US)**
Erfinder: **Zorner, Paul Steffen, Dr.**
**2939, Segoya Way**
**Carlsbad, CA 92009(US)**
Erfinder: **Westphalen, Karl-Otto, Dr.**
**Mausbergweg 58**
**W-6720 Speyer(DE)**

(54) **Azolylpyrimidin- und -triazinderivate und sie enthaltende Mittel.**

(57) Herbizide Mittel, enthaltend mindestens einen herbiziden Wirkstoff aus der Gruppe der Benzothiadiazine I,

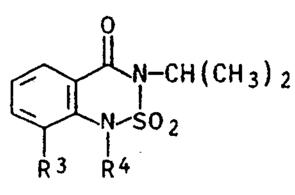

I

wobei die Substituenten folgende Bendeutung haben:
$R^3$ Wasserstoff, Halogen, $C_1$-$C_4$-Alkyl oder $C_1$-$C_4$-Alkoxy und
$R^4$ Wasserstoff oder Cyano
oder dessen umweltverträgliches Salz sowie mindestens ein Azolylpyrimidin IIa oder ein Azolyltriazin IIb

EP 0 421 266 A1

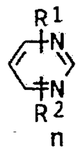 IIa

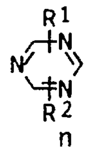 IIb

wobei per Index und die Substituenten folgende Bedeutung haben:

$R^1$ ggf. substituierter fünfgliedriger über sein N-Atom gebundener Heteroaromat, enthaltend zwei oder drei Stickstoffatome;

$R^2$ Halogen; $C_{1-4}$-Alkyl; $C_{1-4}$-Halogenalkyl; Hydroxy; $C_{1}$-$_{4}$-Alkoxy; $C_1$-$C_4$-Halogenalkoxy; $C_1$-$C_4$Alkylthio; $C_1$-$C_4$-Halogenalkylthio; Cyano; Nitro; Amino; $C_1$-$C_4$-Alkylamino; $C_3$-$C_7$-Cycloalkylamino; Di-$C_1$-$C_4$-Alkylamino;
ggf. substituierter Phenyl, Phenoxy, Phenylamino, Benzyl, Benyloxy oder Benzylamino; oder ein Rest $R^1$ und

n 0, 1 oder 2, wobei die Reste $R^2$ in Falle von n = 2 unterschiedlich sin können,

oder deren umweltverträgliche Salze sowie hierfür übliche Zusatzstoffe.

# AZOLYLPYRIMIDIN- UND -TRIAZINDERIVATE UND SIE ENTHALTENDE MITTEL

Die vorliegende Erfindung betrifft herbizide Mittel, enthaltend mindestens einen herbiziden Wirkstoff aus der Gruppe der Benzothiadiazine der Formel I,

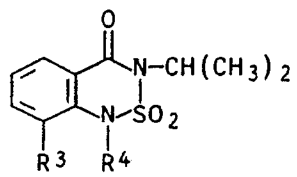

I

in der die Substituenten folgende Bedeutung haben:
$R^3$ Wasserstoff, Halogen, $C_1$-$C_4$-Alkyl oder $C_1$-$C_4$-Alkoxy und
$R^4$ Wasserstoff oder Cyano
oder dessen umweltverträgliches Salz ein Azolylpyrimidin der Formel IIa oder ein Azolyltriazin der Formel IIb

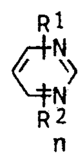

IIa

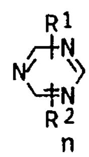

IIb

in denen der Index und die Substituenten folgende Bedeutung haben:
$R^1$ ein fünfgliedriger über sein N-Atom gebundener Heteroaromat, enthaltend zwei oder drei Stickstoffatome, welcher eine Nitrogruppe und/oder ein bis drei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aminocarbonyl, $C_1$-$C_4$-Alkylaminocarbonyl und/oder Di-$C_1$-$C_4$-Alkylaminocarbonyl;
$R^2$ Halogen; $C_1$-$C_4$-Alkyl; $C_1$-$C_4$-Halogenalkyl; Hydroxy; $C_1$-$C_4$-Alkoxy; $C_1$-$C_4$-Halogenalkoxy; $C_1$-$C_4$-Alkylthio; $C_1$-$C_4$-Halogenalkylthio; Cyano; Nitro; Amino; $C_1$-$C_4$-Alkylamino; $C_3$-$C_7$-Cycloalkylamino; Di-$C_1$-$C_4$-Alkylamino;
Phenyl, Phenoxy, Phenylamino, Benzyl, Benyloxy oder Benzylamino, wobei die aromatischen Reste ihrerseits ein bis fünf Halogenatome und/oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder ein Rest $R^1$ und
n 0, 1 oder 2, wobei die Reste $R^2$ im Falle von $n = 2$ auch unterschiedlich sein können,
oder deren umweltverträgliche Salze sowie hierfür übliche Zusatzstoffe und Verfahren zur selektiven Bekämpfung von unerwünschtem Pflanzenwuchs mit diesen Mitteln.

Außerdem betrifft die Erfindung 2-(1-Imidazolyl)pyrimidin II

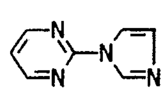

II

und dessen umweltverträgliche Salze.

Aus der EP-A 257 850 sind ein- bis zweifach durch Azolylreste substituierte Pyrimidinderivate als Pharmaka bekannt. Außerdem werden in der US-A 3 308 122 dreifach durch Imidazol substituierte Triazine als Fungizide und in der DE-A 2 051 521 ein- bis zweifach durch Imidazol substituierte Pyrimidine und Triazine als Zusatz für Gummi/Polyesterzusammensetzungen beschrieben. Daneben dienen aminosubstituierte Azolylpyrimidine und -triazine als Pharmaka, Insektizide und Zwischenprodukte (DE-A 3 611 427, DE-A 3 639 563, DE-A 2 736 876, JP-A 49/17675, JP-A 49/17677, JP-A 54/40831 und JP-A 56/55 302).

Herbizide Wirkstoffe aus der Gruppe der Benzothiadiazine der Formel I dienen der Bekämpfung von

unerwünschten Pflanzen (DE-A 1 542 836, US-A 4 158 559).

Der Erfindung lagen Verbindungen als Aufgabe zugrunde, welche in der Lage sind, die Wirkung der bekannten Herbizide gegen unerwünschte Pflanzen zu steigern, ohne daß die Kulturpflanzenverträglichkeit dieser Herbizide verloren geht. Solche Verbindungen werden auch als Synergisten bezeichnet.

Entsprechend dieser Aufgabe wurde 2-(1-imidazolyl)pyrimidin II gefunden. Außerdem wurde gefunden, daß sich die eingangs definierten Azolylpyrimidine und -triazine IIa und IIb als Synergisten bei der Anwendung von Herbiziden aus der Gruppe der Benzothiadiazine I eignen.

Man erhält 2-(1-Imidazolyl)pyrimidin II besonders vorteilhaft, indem man ein 2-Halogenpyrimidin III in einem inerten aprotisch apolaren organischen Lösungsmittel mit einem Alkalimetall- oder Erdalkalimetallsalz von Imidazol IV umsetzt.

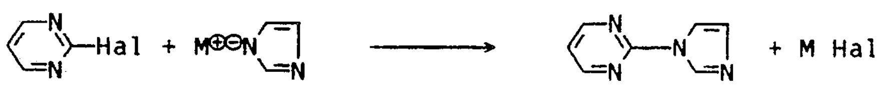

III IV II

Hal in der Formel III bedeutet ein Halogenatom wie Fluor, Chlor, Brom und Jod, vorzugsweise Chlor und Brom; $M^{\oplus}$ in der Formel IV steht für ein Äquivalent eines Alkalimetall- oder Erdalkalimetallkations, wie die Kationen von Lithium, Natrium, Kalium, Magnesium und Kalzium.

Die Umsetzung kann kontinuierlich oder diskontinuierlich bei Normaldruck oder unter Drucken bis 30 bar, vorzugsweise bei Normaldruck und Temperaturen von 40 °C bis 200 °C, vorzugsweise 70 °C bis 160 °C durchgeführt werden.

Als Lösungsmittel eignen sich beispielsweise Ether wie Dioxan, Tetrahydrofuran, Ethylenglycoldimethylether und Dimethylenglycoldiethylether; Alkohole wie Methanol, Ethanol und 2-Propanol; Ketone wie Aceton und Ethylenmethylketon sowie Dimethylformamid, Acetonitril und Dimethylsulfoxid und entsprechende Gemische.

Zur Steigerung der Umsetzungsgeschwindigkeit kann es vorteilhaft sein ein Jodid wie Kaliumjodid zuzugeben.

Die benötigten Salze des Imidazols lassen sich nach allgemeinem Fachwissen herstellen.

Im Hinblick auf die bestimmungsgemäße Verwendung als Synergisten kommen neben 2-(1-Imidazolyl)-pyrimidin II, bevorzugt Azolylpyrimidine und triazine Ia und Ib in Betracht, in denen der Index und die Substituenten folgende Bedeutung haben:

$R^1$ Hetaryl wie Imidazolyl und Triazolyl, vorzugsweise 1-Imidazolyl und 1,2,4-Triazol-1-yl, welches eine Nitrogruppe und/oder ein bis drei der folgenden Reste tragen kann: Halogen wie Fluor, Chlor, Brom und Jod, vorzugsweise Fluor, Chlor und Brom;

Alkyl wie Methyl, Ethyl, Propyl, 1-Methylethyl, Butyl, 1-Methylpropyl, 2-Methylpropyl und 1,1-Dimethylbutyl, vorzugsweise Methyl und Ethyl; Halogenalkyl wie Fluormethyl, Difluormethyl, Trifluormethyl, Chlordifluormethyl, Trichlormethyl, 1-Fluorethyl, 2-Fluorethyl, 2,2-Difluorethyl, 2, 2, 2-Trifluorethyl, 2-Chlor-2,2-difluorethyl, 2,2-Dichlor-2-fluorethyl, 2, 2, 2-Trichlorethyl und Pentafluorethyl, -pyrimidin, vorzugsweise Trifluormethyl und Trichlormethyl;

Alkoxy wie Methoxy, Ethoxy, Propyloxy, 1-Methylethoxyl Butyloxy, 1-Methylpropyloxy, 2-Methylpropyloxy und 1,1-Dimethylethoxy, vorzugsweise Methoxy und Ethoxy;

Halogenalkoxy wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, vorzugsweise Trifluormethoxy;

Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio und Ethylthio;

Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl und 1,1-Dimethylethoxycarbonyl, vorzugsweise Methoxycarbonyl und Ethoxycarbonyl;

Aminocarbonyl;

Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl und 1,1-Dimethylethylaminocarbonyl, vorzugsweise Methylaminocarbonyl;

und/oder

Dialkylaminocarbonyl N,N-Dimethylaminocarbonyl, N,N-Diethylaminocarbonyl, N,N-Dipropylaminocarbonyl,

N,N-Di-(1-Methylethyl)aminocarbonyl, N,N-Dibutylaminocarbonyl, N,N-Di-(1-methylpropyl)aminocarbonyl, N,N-Di-(2-methylpropyl)aminocarbonyl und N,N-Di-(1,1-dimethylethyl)aminocarbonyl, vorzugsweise Dimethylaminocarbonyl;

$R^2$ Halogen wie bei $R^1$ genannt, vorzugsweise Fluor, Chlor und Brom; Alkyl wie bei $R^1$ genannt, vorzugsweise Methyl und Ethyl;

Halogenalkyl wie bei $R^1$ genannt, vorzugsweise Trifluormethyl und Trichlormethyl;

Alkoxy wie im allgemeinen und im besonderen bei $R^1$ genannt;.

Halogenalkoxy wie im allgemeinen und im besonderen bei $R^1$ genannt;

Alkylthio wie im allgemeinen und im besonderen bei $R^1$ genannt;

Halogenalkylthio wie Fluormethylthio, Difluormethylthio, Trifluormethylthio, Chlordifluormethylthio, Dichlorfluormethylthio, Trichlormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2- fluorethylthio, 2,2,2-Trichlorethylthio und Pentafluorethylthio, vorzugsweise Trifluormethylthio; Cyano; Nitro; Amino;

Alkylamino wie Methylamino, Ethylamino, Propylamino, 1-Methylethylamino, Butylamino, 1-Methylpropylamino, 2-Methylpropylamino und 1,1-Dimethylethylamino, vorzugsweise Methylamino und Ethylamino;

Cycloalkylamino wie Cyclopropylamino, Cyclobutylamino, Cyclopentylamino, Cyclohexylamino und Cycloheptylamino, vorzugsweise Cyclopropylamino;

Dialkylamino wie N,N-Dimethylamino, N,N-Diethylamino, N,N-Dipropylamino, N,N-Di-(1-inethylethyl)amino, N,N-Dibutylamino, N,N-Di-(1-methylpropyl)amino, N,N-Di-(2-methylpropyl)amino und N,N-Di-(1,1-dimethylethyl)amino, vorzugsweise Dimethylamino und Diethylamino;

Phenyl, Phenoxy, Phenylamino, Benzyl, Benzyloxy und Benzylamino, wobei die aromatischen Reste ihrerseits ein bis fünf Halogenatome wie bei $R^1$ genannt, vorzugsweise Fluor und Chlor und/oder ein bis drei der folgenden Gruppen tragen können:

Alkyl wie im allgemeinen und im besonderen bei $R^1$ genannt;

Halogenalkyl wie im allgemeinen und im besonderen bei $R^1$ genannt;

Alkoxy wie im allgemeinen und im besonderen bei $R^1$ genannt;

Halogenalkoxy wie im allgemeinen und im besonderen bei $R^1$ genannt;

Alkylthio wie im allgemeinen und im besonderen bei $R^1$ genannt;

oder ein Rest $R^1$ und

n 0, 1 oder 2, vorzugsweise 0 oder 1,

wobei die Reste $R^2$ verschieden sein können wenn n 2 bedeutet.

Spezielle, neben 2-(1-Imidazolyl)pyrimidin II als Synergisten insbesondere bevorzugte Azolylpyrimidinderivate IIa und Azolyltriazinderivate IIb sind in den folgenden Tabellen A und B aufgeführt.

Tabelle A

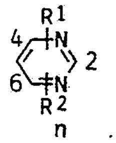 IIa [ $R^1$= 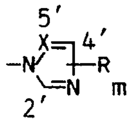 ]

| Pos $R^1$ | X | $R_m$ | $R^2{}_n$ |
|---|---|---|---|
| 2 | CH | H | 4-$R^1$; 6-Cl |
| 2 | CH | H | 4,6-$R^1$, $R^1$ |
| 2 | CH | H | 4,6-Cl, Cl |
| 4 | CH | H | 2,4-Cl, Cl |
| 4 | CH | H | 2-Cl, 6-$R^1$ |
| 2 | CH | H | 4-$CF_3$ |
| 2 | CH | H | 4-Cl, 6-$CH_3$ |
| 4 | CH | H | 2-Cl, 6-$CH_3$ |
| 2 | CH | H | 4-$R^1$, 6-$CH_3$ |
| 2 | CH | H | 4-Cl |
| 4 | CH | H | 2-Cl |
| 2 | CH | H | 4-$R^1$ |
| 2 | $CR_m$ | 5'-$NO_2$ | H |
| 2 | CH | 2'-$CH_3$ | H |
| 2 | $CR_m$ | 4'5'-Cl, Cl | H |
| 2 | CH | 2'-$CO_2CH_3$ | H |
| 2 | CH | 2'-$CH_3$ | H |
| 2 | $CR_m$ | 4'-$CH_3$, 5'-Br | H |
| 2 | CH | 2'-$CONH_2$ | H |
| 2 | $CR_m$ | 2',4',5'-Br,Br,Br | H |
| 2 | CH | H | 4-$CH_3$ |
| 2 | CH | H | 4-OH, 6-$CH_3$ |
| 4 | CH | H | 6-$R^1$ |
| 2 | CH | H | 4-$OCH_3$, 6-$CH_3$ |
| 4 | CH | H | 4-$OCH_3$, 6-$CH_3$ |
| 2 | CH | H | 4-$OCH_3CH_3$, 6-$CH_3$ |
| 2 | CH | H | 4-$OCH_3$ |
| 4 | CH | H | 6-$OCH_3$ |
| 4 | CH | H | 2-$NH_2$, 6-$CH_3$ |

Tabelle A (ff)

| Pos $R^1$ | X | $R_m$ | $R^2{}_n$ |
|---|---|---|---|
| 4 | CH | H | $2-NH_2$, $6-OCH_3$ |
| 2 | CH | H | $4,6-CH_3$, $CH_3$ |
| 2 | CH | H | $4-NH_2$, 6-NH 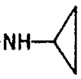 |
| 2 | CH | H | 4-Cl, $6-NH-C_3H_7$ |
| 2 | CH | H | $4,6-[NHC(CH_3)_3]_2$ |
| 2 | CH | $2'-CH_3$ | $4,6-R^1$, $R^1$ |
| 2 | CH | H | 4,6-OH, OH |
| 2 | CH | H | $4-NH_2$, 6-OH |
| 2 | CH | H | $4,6-NH_2$, $NH_2$ |
| 2 | CH | H | $4,6-[NHCH(CH_3)_2]_2$ |
| 2 | CH | H | $4-NHCH(CH_3)_2$, $6-OCH_3$ |
| 2 | CH | H | $4,6-[N(CH_3)_2]_2$ |
| 2 | CH | H | $4,6-(NH-C_2H_5)_2$ |
| 2 | CH | H | $4-NH_2$, $6-OCH_3$ |
| 2 | CH | H | $4-NH_2$, $6-CH_3$ |
| 2 | CH | $2'-CH_3$ | $4,6-NH_2$, $NH_2$ |
| 2 | CH | $2'-CH_3$, $4'-C_2H_5$ | $4,6-NH_2$, $NH_2$ |
| 2 | CH | H | 4-Cl, $6-NHCH_2CH(CH_3)_2$ |
| 2 | CH | H | $4,6-CH_2CH_3$, $CH_2CH_3$ |
| 2 | CH | H | $4-C_6H_5$ |
| 4 | CH | H | $2-NH_2$, $6-OCH_2CH_3$ |
| 4 | CH | H | $2-NH_2$, 6-Cl |
| 4 | CH | H | $2-NHCH_3$, $6-CH_3$ |
| 4 | CH | H | $2-NHCH_3$, $6-OCH_3$ |
| 2 | N | H | $4-R^1$; 6-Cl |
| 2 | N | H | $4,6-R^1$, $R^1$ |
| 2 | N | H | 4,6-Cl, Cl |
| 4 | N | H | 2,4-Cl, Cl |
| 4 | N | H | 2-Cl, $6-R^1$ |
| 2 | N | H | $4-CF_3$ |
| 2 | N | H | 4-Cl, $6-CH_3$ |
| 4 | N | H | 2-Cl, $6-CH_3$ |

Tabelle A (ff)

| Pos $R^1$ | X | $R_m$ | $R^2_n$ |
|---|---|---|---|
| 2 | N | H | 4-$R^1$, 6-$CH_3$ |
| 2 | N | H | 4-Cl |
| 4 | N | H | 2-Cl |
| 2 | N | H | 4-$R^1$ |
| 2 | N | 4'-$NO_2$ | H |
| 2 | N | 2'-$CH_3$ | H |
| 2 | N | 2',4'-Cl,Cl | H |
| 2 | N | 2'-$CO_2CH_3$ | H |
| 2 | N | 2'-$CH_3$ | H |
| 2 | N | 2'-Br, 4'-$CH_3$ | H |
| 2 | N | 2'-$CONH_2$ | H |
| 2 | N | 2',4'-Br, Br | H |
| 2 | N | H | 4-$CH_3$ |
| 2 | N | H | 4-OH, 6-$CH_3$ |
| 4 | N | H | 6-$R^1$ |
| 2 | N | H | 4-$OCH_3$, 6-$CH_3$ |
| 4 | N | H | 2-$OCH_3$, 6-$CH_3$ |
| 2 | N | H | 4-$OCH_2CH_3$, 6-$CH_3$ |
| 2 | N | H | 4-$OCH_3$ |
| 4 | N | H | 6-$OCH_3$ |
| 4 | N | H | 2-$NH_2$, 6-$CH_3$ |
| 4 | N | H | 2-$NH_2$, 6-$OCH_3$ |
| 2 | N | H | 4,6-$CH_3$, $CH_3$ |
| 2 | N | H | 4-$NH_2$, 6-NH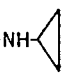 |
| 2 | N | H | 4-Cl, 6-NH-$C_3H_7$ |
| 2 | N | H | 4,6-$[NHC(CH_3)_3]_2$ |
| 2 | N | 2'-$CH_3$ | 4,6-$R^1$, $R^1$ |
| 2 | N | H | 4,6-OH, OH |
| 2 | N | H | 4-$NH_2$, 6-OH |
| 2 | N | H | 4,6-$NH_2$, $NH_2$ |
| 2 | N | H | 4,6-$[NHCH(CH_3)_2]_2$ |
| 2 | N | H | 4-$NHCH(CH_3)_2$, 6-$OCH_3$ |
| 2 | N | H | 4,6-$[N(CH_3)_2]_2$ |

Tabelle A (ff)

| Pos $R^1$ | X | $R_m$ | $R^2{}_n$ |
|---|---|---|---|
| 2 | N | H | 4,6-$(NH-C_2H_5)_2$ |
| 2 | N | H | 4-$NH_2$, 6-$OCH_3$ |
| 2 | N | H | 4-$NH_2$, 6-$CH_3$ |
| 2 | N | 2'-$CH_3$ | 4,6-$NH_2$, $NH_2$ |
| 2 | N | 2'-$CH_3$, 4'-$C_2H_5$ | 4,6-$NH_2$, $NH_2$ |
| 2 | N | H | 4-Cl, 6-$NHCH_2(CH_3)_2$ |
| 2 | N | H | 4,6-$CH_2CH_3$, $CH_2CH_3$ |
| 2 | N | H | 4-$C_6H_5$ |
| 4 | N | H | 2-$NH_2$, 6-$OCH_2CH_3$ |
| 4 | N | H | 2-$NH_2$, 6-Cl |
| 4 | N | H | 2-$NHCH_3$, 6-$CH_3$ |
| 4 | N | H | 2-$NHCH_3$, 6-$OCH_3$ |

Tabelle B

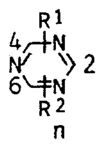

IIb

[$R^1$= 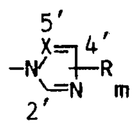 ]

| Pos $R^1$ | X | $R_m$ | $R^2{}_n$ |
|---|---|---|---|
| 2 | CH | H | 4-$R^1$; 6-Cl |
| 2 | CH | H | 4,6-$R^1$, $R^1$ |
| 2 | CH | H | 4,6-Cl, Cl |
| 2 | CH | H | 4-$CF_3$ |
| 2 | CH | H | 4-Cl, 6-$CH_3$ |
| 2 | CH | H | 4-$R^1$, 6-$CH_3$ |
| 2 | CH | H | 4-Cl |
| 2 | CH | H | 4-$R^1$ |
| 2 | $CR_m$ | 5'-$NO_2$ | H |
| 2 | CH | 2'-$CH_3$ | H |
| 2 | $CR_m$ | 4',5'-Cl, Cl | H |
| 2 | CH | 4'-$CO_2CH_3$ | H |

Tabelle B (ff)

| Pos $R^1$ | X | $R_m$ | $R^2{}_n$ |
|---|---|---|---|
| 2 | CH | 4'-$CH_3$ | H |
| 2 | $CR_m$ | 4'-$CH_3$, 5'-Br | H |
| 2 | CH | 4'-$CONH_2$ | H |
| 2 | $CR_m$ | 2',4',5'-Br,Br,Br | H |
| 2 | CH | H | 4-$CH_3$ |
| 2 | CH | H | 4-OH, 6-$CH_3$ |
| 2 | CH | H | 4-$OCH_3$, 6-$CH_3$ |
| 2 | CH | H | 4-$OCH_2CH_3$, 6-$CH_3$ |
| 2 | CH | H | 4-$OCH_3$ |
| 2 | CH | H | 4-$NH_2$, 6-$CH_3$ |
| 2 | CH | H | 4-$NH_2$, 6-$OCH_3$ |
| 2 | CH | H | 4,6-$CH_3$, $CH_3$ |
| 2 | CH | H | 4-$NH_2$, 6-NH 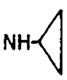 |
| 2 | CH | H | 4-Cl, 6-NH-$C_3H_7$ |
| 2 | CH | H | 4,6-$[NHC(CH_3)_3]_2$ |
| 2 | CH | 2'-$CH_3$ | 4,6-$R^1$, $R^1$ |
| 2 | CH | H | 4,6-OH, OH |
| 2 | CH | H | 4-$NH_2$, 6-OH |
| 2 | CH | H | 4,6-$NH_2$, $NH_2$ |
| 2 | CH | H | 4,6-$[NHCH(CH_3)_2]_2$ |
| 2 | CH | H | 4-$NHCH(CH_3)_2$, 6-$OCH_3$ |
| 2 | CH | H | 4,6-$[N(CH_3)_2]_2$ |
| 2 | CH | H | 4,6-$(NH-C_2H_5)_2$ |
| 2 | CH | 2'-$CH_3$ | 4,6-$NH_2$, $NH_2$ |
| 2 | CH | 2'-$CH_3$,4'-$C_2H_5$ | 4,6-$NH_2$, $NH_2$ |
| 2 | CH | H | 4-Cl, 6-$NHCH_2CH(CH_3)_2$ |
| 2 | CH | H | 4,6-$CH_2CH_3$, $CH_2CH_3$ |
| 2 | CH | H | 4-$C_6H_5$ |
| 2 | CH | H | 4-$NH_2$, 6-$OCH_2CH_3$ |
| 2 | CH | H | 4-$NH_2$, 6-Cl |
| 2 | CH | H | 4-$NHCH_3$, 6-$CH_3$ |
| 2 | CH | H | 4-$NHCH_3$, 6-$OCH_3$ |

Tabelle B (ff)

| Pos $R^1$ | X | $R_m$ | $R^2_n$ |
|---|---|---|---|
| 2 | N | H | 4-$R^1$, 6-Cl |
| 2 | N | H | 4,6-$R^1$, $R^1$ |
| 2 | N | H | 4,6-Cl, Cl |
| 2 | N | H | 4-$CF_3$ |
| 2 | N | H | 4-Cl, 6-$CH_3$ |
| 2 | N | H | 4-$R^1$, 6-$CH_3$ |
| 2 | N | H | 4-Cl |
| 2 | N | H | 4-$R^1$ |
| 2 | N | 4'-$NO_2$ | H |
| 2 | N | 2'-$CH_3$ | H |
| 2 | N | 2'-4'-Cl, Cl | H |
| 2 | N | 2'-$CO_2CH_3$ | H |
| 2 | N | 2'-$CH_3$ | H |
| 2 | N | 2'-Br, 4'-$CH_3$ | H |
| 2 | N | 4'-$CONH_2$ | H |
| 2 | N | 2',4'-Br, Br | H |
| 2 | N | H | 4-$CH_3$ |
| 2 | N | H | 4-OH, 6-$CH_3$ |
| 2 | N | H | 4-$OCH_3$, 6-$CH_3$ |
| 2 | N | H | 4-$OCH_2CH_3$, 6-$CH_3$ |
| 2 | N | H | 4-$OCH_3$ |
| 2 | N | H | 4-$NH_2$, 6-$CH_3$ |
| 2 | N | H | 4-$NH_2$, 6-$OCH_3$ |
| 2 | N | H | 4,6-$CH_3$, $CH_3$ |
| 2 | N | H | 4-$NH_2$, 6-NH 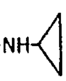 |
| 2 | N | H | 4-Cl, 6-NH-$C_3H_7$ |
| 2 | N | H | 4,6-$[NHC(CH_3)_3]_2$ |
| 2 | N | 2'-$CH_3$ | 4,6-$R^1$, $R^1$, |
| 2 | N | H | 4,6-OH, OH |
| 2 | N | H | 4-$NH_2$, 6-OH |
| 2 | N | H | 4,6-$NH_2$, $NH_2$ |

Tabelle B (ff)

| Pos $R^1$ | X | $R_m$ | $R^2_n$ |
|---|---|---|---|
| 2 | N | H | $4,6-[NHCH(CH_3)_2]_2$ |
| 2 | N | H | $4-NHCH(CH_3)_2$, $6-OCH_3$ |
| 2 | N | H | $4,6-[N(CH_3)_2]_2$ |
| 2 | N | H | $4,6-(NH-C_2H_5)_2$ |
| 2 | N | $2'-CH_3$ | $4,6-NH_2$, $NH_2$ |
| 2 | N | $2'-CH_3$, $4'-C_2H_5$ | $4,6-NH_2$, $NH_2$ |
| 2 | N | H | 4-Cl, $6-NHCH_2CH(CH_3)_2$ |
| 2 | N | H | $4,6-CH_2CH_3$, $CH_2CH_3$ |
| 2 | N | H | $4-C_6H_5$ |
| 4 | N | H | $4-NH_2$, $6-OCH_2CH_3$ |
| 2 | N | H | $4-NH_2$, 6-Cl |
| 2 | N | H | $4-NHCH_3$, $6-CH_3$ |
| 2 | N | H | $4-NHCH_3$, $6-OCH_3$ |

Spezielle Beispiele für herbizide Benzothiadiazine I, deren Wirkung durch 2-(1-Imidazolyl)pyrimidin II und die synergistischen Azolylpyrimidin- bzw. triazinderivate IIa und IIb verbessert werden kann, sind in der folgenden Tabelle C aufgeführt:

Tabelle C

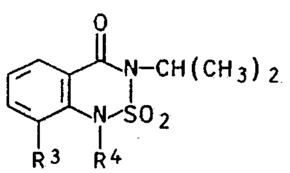

I

| Nr. | $R^3$ | $R^4$ | Literatur |
|---|---|---|---|
| I. 001 | H | H | DE-A 1 542 836 |
| I. 002 | F | H | DE-A 2 444 383 |
| I. 003 | Cl | H | DE-A 2 444 383 |
| I. 004 | $CH_3$ | H | DE-A 2 443 901 |
| I. 005 | H | Na | DE-A 1 542 836 |
| I. 006 | F | Na | DE-A 2 444 383 |
| I. 007 | Cl | Na | DE-A 2 444 383 |
| I. 008 | $CH_3$ | Na | DE-A 2 443 901 |
| I. 009 | H | CN | DE-A 2 656 289 |
| I. 010 | F | CN | DE-A 2 656 289 |
| I. 011 | Cl | CN | DE-A 2 656 289 |
| I. 012 | $CH_3$ | CN | DE-A 2 656 289 |

Für herbizide Benzothiadiazine I werden unterschiedliche Mengen einer synergistisch wirkenden Verbindung benötigt, wenn das Herbizid in verschiedenen Kulturen eingesetzt wird. Die Mengenverhältnisse sind dabei in breiten Bereichen variabel. Sie sind außerdem abhängig von der Struktur der Benzothiadiazine II und der jeweiligen Zielkultur. Geeignete Anteilsverhältnise Syngergist : Herbizid liegen bei 0,01 : 1, bis 10 : 1 vorzugsweise bei 0,05 : 1 bis 6 : 1 und insbesondere bei 0,1 : 1 bis 4 : 1 Gew.-Teile.

Der herbizide Wirkstoff und die synergistisch wirkende Verbindung können gemeinsam oder getrennt nach dem Auflaufen auf die Blätter und Sprosse der Kulturpflanzen und der unerwünschten Pflanzen ausgebracht werden. Bevorzugt wird das synergistisch wirkende Mittel gleichzeitig mit dem herbiziden Wirkstoff ausgebracht. Auch eine getrennt Ausbringen, wobei der Synergist zuerst und anschließend der herbizide Wirkstoff auf das Feld gebracht werden, ist möglich. Herbizider Wirkstoff und Synergist können hierbei als Spritzmittel in suspendierbarer, emulgierbarer oder löslicher Form gemeinsam oder getrennt formuliert vorliegen.

Möglich ist auch eine Behandlung der Kulturpflanzensamen mit dem Synergisten vor der Aussaat. Der herbizide Wirkstoff wird dann allein in der üblichen Weise appliziert.

Die erforderlichen Aufwandmengen an reiner Wirkstoffmenge, d. h. ohne Formulierungshilfsmittel ist abhängig von der Zusammensetzung des Pflanzenbestandes, vom Entwicklungsstadium der Pflanzen, von den klimatischen Verhältnissen am Einsatzort sowie von der Anwendungstechnik. Im allgemeinen betragen die Aufwandmengen 0,25 bis 5 kg, vorzugsweise 0,5 bis 2,5 kg Wirkstoff/ha.

Als Kulturen, in denen die erfindungsgemäßen herbiziden und pflanzenschützenden Mittel angewandt werden können, kommen im wesentlichen diejenigen in Betracht, in denen auch die Einzelwirkstoffe der Mischung eingesetzt werden können. Im Falle von Benzothiadiazinonderivate der Formel II enthaltenden Mittel sind dies beispielsweise Gerste, Kultursorghum, Mais, Reis, Weizen, Erbsen, Erdnüsse, Soja und Kulturrasen.

Von Bedeutung ist ferner die Ausbringungstechnik. Werden die neuen Mittel zum Zwecke der Bekämpfung unerwünschter Pflanzen bei Kulturpflanzen, die eine ungenügende Verträglichkeit aufweisen, eingesetzt, so wird nach speziellen Methoden die Ausbringung so gelenkt, daß die Blätter der Kulturplanzen möglichst wenig mit den Mitteln in Berühung kommen, während die dazwischen oder darunter wachsenden unerwünschten Pflanzen oder die freie Bodenfläche von den Mitteln getroffen werden (Unterblattspritzung, post-directed, lay-by).

Die neuen herbiziden Mittel können neben dem Azolylderivat der Formel IIa und/oder IIb als Synergist und dem Herbizid aus der Gruppe der Benzothiadiazine I weitere herbizide und wachstumsregulierende Wirkstoffe andere inerte Zusätze enthalten.

Heben der synergistischen Wirkung bei der gemeinsamen Anwendung mit Benzothiadiazinen der Formel I können 2-(1-Imidazolyl)pyrimidin II und die Azolylpyrimidine und -trazine IIa und IIb auch bei folgenden Herbiziden als Synergisten eingesetzt werden (Handelsnamen in Klammern)

- 5-Amino-4-chlor-2-phenylpyridazin-3(2H)-on (Pyrazon)
- 4-Chlor-5-methylamino-2-(trifluormethylphenyl)-3(2H)-pyridazin-3-(2H)-on (Monometfluorazon)
- 3-(3-Chlor-4-methylphenyl)-1,1-dimethylharnstoff (Chlortoluron)
- 3-(4-Bromphenyl)-1-methoxy-1-methylharnstoff (Metobromuron)
- 3-(4-Isopropylphenyl)-1,1-dimethylharnstoff (Isoproturon)
- 3-(3,4-Dichlorphenyl)1-methoxy-1-methylharnstoff (Linuron)
- 3-(3,4-Dichlorphenyl)-1,1-dimethylharnstoff (Diuron)
- 3-(2-Benzothiazolyl)1,3-dimethylharnstoff (Methabenzthiazuron)
- 1,1-Dimethyl-3-(3-trifluormethylphenyl)-harnstoff (Fluometuron)
- 2-[3-(4-Methoxy-6-methyl-1,3,5-triazin-2-yl)aminocarbonylaminosulfonyl]benzsäure)methylester (Metsulfuron-methyl)
- 2-[3-(4,6-dimethoxypyrimidin-2-yl)aminocarbonylaminosulfonyl]benzoesäuremethylester (Bensulfuron-methyl)
- 2-[3-(4-Chlor-6-methoxypyrimidin-2-yl)aminocarbonylaminosulfonyl]benzoesäureethylester (Chlorimuron)
- 2-[3-(4-Methoxy-6-methyl-1,3,5-triazin-2-yl)-3-methylaminocarbonylaminosulfonyl]benzoesäuremethylester
- 3-(4,6-Dimethoxy-1,3,5-triazin-2-yl)-1-[2-(2-methoxyethoxy)phenylsulfonyl]harnstoff (Cinosulfuron)
- 2-[3-(4,6-bis(difluormethoxy)pyrimidin-2-yl)-aminocarbonylaminosulfonyl]benzoesäuremethylester-(Primisulfuron)
- 2-(2-Chlorethoxy)N-[[(4-methoxy-6-methyl-1,3,5-triazin-2-yl)aminocarbonyl]benzolsulfonsäureamid (Triasulfuron)
- 2-[[(4,6-Dimethoxypyrimidin-2-yl)aminocarbonyl]aminosulfonyl]-3-pyridincarbonsäure-N,N-dimethylamid
- S-(4-Chlorbenzyl)-N,N-diethylthiocarbamat (Benthiocarb)
- S-Benzyl-N,N-dipropylthiocarbamat (Prosulfocarb)
- S-Ethyl-N,N,-di-iso-butylthiocarbamat (Butylat)

- S-EthylN,N-di-n-propylthiocarbamat (EPTC)
- 3-(Methoxycarbonylamino)phenyl-N-(3-methylphenyl)carbamat (Phenmedipham)
- 3-(Ethoxycarbonylamino)phenyl-N-phenylcarbamat (Desmedipham)
- Isopropyl-N-(3-chlorphenyl)-carbamat (Chloropropham)
- 2,6-Dinitro-N,N-dipropyl-4-trifluormethylanilin (Trifluralin)
- 3,4-Dimethyl-2,6-Dinitro-N-1-ethylpropyl-anilin (Pendimenthalin)
- 4-Amino-3-methyl-6-phenyl 1,2,4-triazin-5(4H)-on (Metamitron)
- 4-Amino-6-tert.-butyl-3-methylthio-1,2,4-triazin-5(4H)-on (Metribuzin) 2-(2-Chlor-4-ethylamino-1,3,5-triazin-yl-amino)-2-methylpropionitril
- (Cyanazin)
2-Chlor-4-ethylamino-6-iso-propylamino-1,3,5-triazin (Atrazin)
- 2-Chlor-4-ethylamino-6-tert.-butylamino- -1,3,5-triazin
- (Terbutylazin)
3-Chlor-4-chromethyl-1-(3-trifluormethylphenyl)pyrrolidin-2-on
- (Fluorochloridin)
2-Chlor-6-nitro-3-phenoxyanilin (Aclonifen)
- 3,6-Dichlor-2-methoxybenzoesäure (Dicamba)
-2,5-Dichlor-3-aminobenzoesäure (Amiben)
- 2,4-Dichlorphenoxyessigsäure (2,4-D)
- 2-(2,4-Dichlorphenoxy)propionsäure (Dichloprop)
- 2-(4-Chlor-2-methylphenoxy)propionsäure (Mecoprop)
- 2-[4-(2,4-Dichlorphenoxy)-phenoxy]propionsäuremethylester (Diclofop-methyl)
- 2-[4-(6-Chlor-2-benzoxazolyloxy)phenoxy]propionsäureethylester (Fenoxaprop-ethyl)
- 2-[4-(6-Chlor-2-chinoxanyloxy)phenoxy]propionsäureethylester (Quizalafop-ethyl)
- 2-[4-(3-Chlor-5-trifluormethyl-2-pyridyloxy)phenoxy]propionsäuremethylester (Haloxyfop-methyl)
- 2-[4-(5-Trifluormethyl-2-pyridyloxy)phenoxy]propionsäurebutylester (Fluazifop-bentyl)
4-Amino-3,5-dichlor-6-fluor-2-pyridinyloxyessigsäure, dessen Salze und Ester, z.B. 1-Methylheptylester (Fluroxypyr)
- 7-Chlor-3-methyldinolin-8-carbonsäure (Quinmerac)
- 3,7-Dichlorchinolin-8-carbon-säure (Quinclorac)
- N-(2,4-Difluorphenyl)-2-(3-trifluormethylphenoxy)-3-pyridincarbonsäureamid (Diflufanican)
- exo-1-Methyl-4-(1-methylethyl)-2-(2-methylphenylmethoxy)-7-oxabicyclo(2.2.1)heptan (Cinmethlin)
- 2-(2-Chlorphenyl)methyl-4,4-dimethyl-3-is-oxxazolidinon (Clomazon)
- 5-Methylamino-2-phenyl-4-(3-trifluormethylphenyl)furan-3(2H)-on (Flurtamon)
- 2-[4,5-Dihydro-4-methyl-4-(1-methylethyl)-5-oxo-1H-imidazol2-yl]-5-ethyl-3-pyridin-carbonsäure (Imazethapyr)
- 2-[4,5-Dihydro-4-methyl-4-(1-methylethyl)-5-oxo-1H-imidazol-2-yl)-3-chinolincarbonsäure (Imazaquin)
- 4-Chlor-2-oxobenzothiazolin-3-ylessigsäure (Benazolin)
- 2-Phenyl-3,1-benzoxazin-4-on
- 5-Fluor-2-phenyl-4H-3,1-benzoxazin-4-on (Fluorobentranil)
- 3′,4′-Dichlorpropionanilid (Propanil) - 5- [2-Chlor-4-(trifluormethyl)phenoxyx]-2-nitrobenzoesäure-natriumsalz (Acifluorfen)
- 5-(2,4-Dichlorphenoxy)-2-nitrobenzoesäure-methylester (Bifenox)
- 5-(2-Chlor-4-trifluormethylphenoxy)-2-nitro-N-methansulfonylbenzoesäureamid (Fomesafen)
- 3,5-Dibrom-4-hydroxybenzonitrll (Bromoxynil)
- 3,5 Dijod-4-hydroxybenzonitril (loxynil)

Außerdem ist es nützlich, die erfindungsgemäßen Mischungen auch noch mit weiteren Pflanzenschutzmitteln gemeinsam auszubringen, beispielsweise mit Mitteln zur Bekämpfung von Schadlingen oder phytopathogenen Pilzen bzw. Bakterien. Von Interesse ist ferner die Mischbarkeit mit Mineralstofflösungen, welche zur Behebung von ernährungs- und Spurenelementmängeln eingesetzt werden.

Die erfindungsgemäßen Mittel bzw. bei getrennter Ausbringung die herbiziden Wirkstoffe oder der Synergist werden beispielsweise in Form von direkt versprühbaren Lösungen, Pulvern, Suspensionen, auch hochprozentigen wäßrigen, öligen oder sonstigen Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Die Formulierungen enthalten 0,1 bis 95 Gew.-%, vorzugsweise 0,5 bis 90 Gew.-% der Wirkstoffmischung. Sie können nach an sich bekannten Methoden durchgeführt werden.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch das herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkal- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Hepta decanole, Octadecanole, Salze von sulfatierten Fettalkoholglykolethern, Kondensationsprodukte von sulfoniertem Naphthalin und Naphtalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die erfindungsgemäßen herbiziden und pflanzenschützenden Mittel können zusätzlich mit zahlreichen Vertretern weiterer herbizider oder wachstumsregulierender Wirkstoffgruppen gemischt und gemeinsam angebracht werden.

Herstellungsbeispiel

2-1-Imidazolylpyrimidin

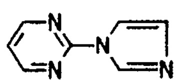

Eine Mischung aus 127.5 g Kaliumhydroxid und 1,0 l Ethanol wurde bei 25° C mit einer Lösung aus 136,2 g (2,0 mol) Imidazol und 1,5 l Ethanol versetzt. Nach zwei weiteren Stunden bei dieser Temperatur wurde das Lösungsmittel bei vermindertem Druck entfernt. Der so erhaltene Rückstand wurde in 1,5 l Dimethylformamid gelöst, und diese Lösung wurde nacheinander zuerst mit 10 g Kaliumjodid und anschließend mit einer Lösung aus 229 g (2,0 mol) 2-Chlorpyrimidin und 1,0 l Dimethylformamid versetzt. Das so erhaltene Reaktionsgemisch wurde zwei Stunden bei 100° C belassen und anschließend von festen Bestandteilen abfiltriert. Aus dem Filtrat wurde das Produkt als Feststoff isoliert.

Fp 128 - 130° C; Ausbeute 280 g (96 %); Wirkstoffbeispiel 1.001.

Die in den nachstehenden Tabellen aufgeführten Verbindungen lassen sich unter entsprechender Abwandlung der Edukte analog zum Synthesebeispiel oder gemäß der zitierten Literatur herstellen.

Tabelle 1

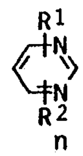

IIa

[ $R^1$ = 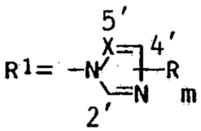 ]

| Nr. | Pos $R^1$ | X | $R_m$ | $R^2_n$ | phys. Daten | Literatur |
|---|---|---|---|---|---|---|
| 1.001 | 2 | CH | H | H | 128 - 130°C | - |
| 1.002 | 2 | CH | H | 4-Cl, 6-$CH_3$ | 90 - 92°C | - |
| 1.003 | 4 | CH | H | 2-Cl, 6-$CH_3$ | 98 - 99°C | - |
| 1.004 | 2 | CH | H | 4-$R^1$, 6-$CH_3$ | 179 - 180°C | - |
| 1.005 | 2 | N | H | 4-Cl | 132 - 134°C | - |
| 1.006 | 2 | N | H | 4,6-Cl, Cl | 93 - 94°C | - |
| 1.007 | 4 | N | H | 2,6-Cl, Cl | 99 - 101°C | - |
| 1.008 | 2 | N | H | 4-$R^1$, 6-Cl | 202 - 204°C | - |
| 1.009 | 2 | N | H | 4-Cl, 6-$CH_3$ | 143 - 145°C | - |
| 1.010 | 4 | N | H | 2-Cl, 6-$CH_3$ | 99 - 101°C | - |
| 1.011 | 2 | N | H | 4-$R^1$, 6-$CH_3$ | 216 - 218°C | - |
| 1.012 | 2 | CH | 5'-$NO_2$ | H | 186 - 188°C | - |
| 1.013 | 2 | CH | 5'-$CH_3$ | H | 96 - 98°C | - |
| 1.014 | 2 | CH | 4',5'-Cl,Cl | H | 145 - 147°C | - |

Tabelle 1 (ff)

| Nr. | Pos $R^1$ | X | $R_m$ | $R^2_n$ | phys. Daten* | Literatur |
|---|---|---|---|---|---|---|
| 1.015 | 2 | CH | 4'-$CO_2CH_3$ | H | 182 - 183°C | - |
| 1.016 | 2 | CH | 4'-$CH_3$ | H | 103 - 104°C | - |
| 1.017 | 2 | CH | 4'-$CONH_2$ | H | >230°C | - |
| 1.018 | 2 | N | H | H | - | 1 |
| 1.019 | 2 | N | H | 4-$CH_3$ | - | 1 |
| 1.020 | 2 | N | H | 4-OH, 6-$CH_3$ | - | 1 |
| 1.021 | 4 | CH | H | 6-$R^1$ | - | 2 |
| 1.022 | 2 | CH | H | 4-$R^1$ | - | 2 |
| 1.023 | 2 | CH | H | 4-$OCH_3$, 6-$CH_3$ | - | 2 |
| 1.024 | 2 | N | H | 4-$OCH_3$, 6-$CH_3$ | - | 2 |
| 1.025 | 4 | N | H | 2-$OCH_3$, 6-$CH_3$ | - | 2 |
| 1.026 | 2 | CH | H | 4-$OCH_2CH_3$, 6-$CH_3$ | - | 2 |
| 1.027 | 4 | CH | H | 2-Cl | - | 2 |
| 1.028 | 2 | CH | H | 4-Cl | - | 2 |
| 1.029 | 4 | CH | H | 6-$OCH_3$ | - | 2 |
| 1.030 | 4 | N | H | 2-$NH_2$, 6-$CH_3$ | - | 3 |
| 1.031 | 4 | CH | H | 2-$NH_2$, 6-$CH_3$ | - | 3 |
| 1.032 | 4 | CH | H | 2-$NH_2$, 6-$OCH_3$ | - | 3 |
| 1.033 | 2 | CH | H | 4,6-$CH_3$, $CH_3$ | - | 4 |

*Fp (°C); NMR ($\delta$ in ppm)

Tabelle 1 (ff)

| Nr. | Pos $R^1$ | X | $R_m$ | $R^2_n$ | phys. Daten* | Literatur |
|---|---|---|---|---|---|---|
| 1.034 | 4 | N | H | 2-$NH_2$, 6-$OCH_3$ | - | 3 |
| 1.035 | 4 | N | H | 2-$NH_2$, 6-$OCH_2CH_3$ | - | 3 |
| 1.036 | 4 | N | H | 2-$NH_2$, 6-Cl | - | 3 |
| 1.037 | 4 | N | H | 2-$NHCH_3$, 6-$CH_3$ | - | 3 |
| 1.038 | 4 | N | H | 2-$NHCH_3$, 6-$OCH_3$ | - | 3 |
| 1.039 | 4 | CH | H | 2-$NH_2$, 6-$OCH_2CH_3$ | - | 3 |
| 1.040 | 4 | CH | H | 2-$NH_2$, 6-Cl | - | 3 |

Tabelle 2

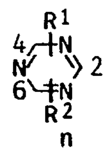 Ib [ $R^1$= 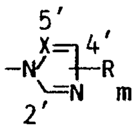 ]

| Nr. | Pos $R^1$ | X | $R_m$ | $R^2_n$ | phys. Daten* | Literatur |
|---|---|---|---|---|---|---|
| 2.001 | 2 | CH | H | 4-$NH_2$, 6-NH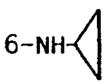 | - | 5 |
| 2.002 | 2 | CH | H | 4-Cl, 6-$NHC_3H_7$ | - | 6 |
| 2.003 | 2 | CH | H | 4,6-$[NHC(CH_3)_3]_2$ | - | 7 |
| 2.004 | 2 | CH | 2'-$CH_3$ | 4,6-$R^1$, $R^1$ | - | 8 |
| 2.005 | 2 | CH | H | 4,6-OH, OH | - | 9 |
| 2.006 | 2 | CH | H | 4,6-$R^1$, $R^1$ | - | 8,10 |
| 2.007 | 2 | CH | H | 4-$NH_2$, 6-OH | - | 9 |
| 2.008 | 2 | CH | H | 4,6-$NH_2$, $NH_2$ | - | 11 |
| 2.009 | 2 | CH | H | 4,6-$[NHCH(CH_3)_2]_2$ | - | 12 |
| 2.010 | 2 | CH | H | 4-$NHCH(CH_3)_2$, 6-$OCH_3$ | - | 12 |
| 2.011 | 2 | CH | H | 4,6-$[N(CH_3)_2]_2$ | - | 12 |
| 2.012 | 2 | CH | H | 4,6-$[NHCH_2CH_3]_2$ | - | 12 |

*Fp (°C); NMR (δ in ppm)

Tabelle 2 (ff)

| Nr. | Pos $R^1$ | X | $R_m$ | $R^2_n$ | phys. Daten | Literatur |
|---|---|---|---|---|---|---|
| 2.013 | 2 | CH | H | 4-$NH_2$, 6-$OCH_3$ | - | 3 |
| 2.014 | 2 | N | H | 4-$NH_2$, 6-$CH_3$ | - | 13 |
| 2.015 | 2 | CH | 2'-$CH_3$ | 4,6-$NH_2$, $NH_2$ | - | 13 |
| 2.016 | 2 | CH | 2'-$CH_3$, 4'-$CH_2CH_3$ | 4,6-$NH_2$, $NH_2$ | - | 13 |
| 2.017 | 2 | CH | H | 4-Cl, 6-$NHCH_2CH(CH_3)_2$ | - | 14 |
| 2.018 | 2 | N | H | 4-$NH_2$, 6-$OCH_3$ | - | 3 |
| 2.019 | 2 | N | H | 4-$NHCH_3$, 6-$OCH_3$ | - | 3 |
| 2.020 | 2 | CH | H | 4-$NH_2$, 6-$CH_3$ | - | 3 |

Literaturangaben:

1) TAKEDA KENKYUSHO 22, 27-46 (1963), zitiert nach CA 60: 12 011 a/b

2) EP-A 257 850
3) DE-A 3 639 563
4) J. Org. Chem. 43, 3957 (1978)
5) DE-A 2 736 876
6) Dokl, Akad. Nauk SSR, 228 (6), 1358-61
7) DE-A 3 611 427
8) US-A 3 308 122
9) JP 49/17675 (2. Mai 1975)
10) Science (Washington, D.C.) 158 (3807), 1462-3 (1967)
11) JP 49/17677 (2. Mai 1974)
12) JP 56/55302 (15. Mai 1981)
13) JP 54/40831 A 79/40831 v (31. Okt. 1979)
14) DE-A 2 051 521

Anwendungsbeispiele

Die synergistische Wirkung der Azolylpyrimidine und -triazine der Formeln IIa und IIb ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei der Vorauflaufbehandlung wurden die in Wasser suspendierten oder emulgierten Wirkstoffe mittels fein verteilender Düsen direkt danach aufgebracht. Anschließend wurden die Gefäße leicht beregnet, um die Keimung und das Wachstum zu fördern. Die so vorbereiteten Gefäße wurden mit durchsichtigen Plastikhauben abgedeckt, bis die Pflanzen angewachsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmenge für die Nachauflaufbehandlung beträgt 0,125 bis 2,5 kg/ha.

Die Testpflanzen wurden entsprechend ihren natürlichen Bedürfnissen bei Temperaturen von 20 bis 35° C bzw. 10 bis 25° C 2 bis 4 Wochen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen: Amaranthus retroflexus, Solanum nigrum, Cassia tora, Glycine max. und Zea mays.

Folgende Wirkstoffe bzw. entsprechende Wirkstoffgemische wurden bei den Versuchen eingesetzt:

Als Benzothiadiazon I diente die Verbindung I. 005.

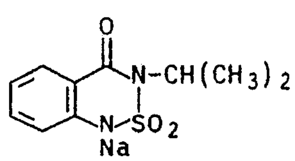

I.005

Der Wirkstoff wurde als wäßrige Lösung in einer Konzentration von 480 g/l formuliert.

Als Vertreter der synergistischen Azolylpyrimidine IIa diente der Wirkstoff 2-(1-Imidazolyl)pyrimidin (1.001). Er wurde als 10 %iges Emuulsionskonzentrat in einem System aus 70 % Lösungsmittel, 20 % Emulgator und 10 % Tensid formuliert. Zusätzlich enthielten alle Formulierungen oberflächenaktive Zusätze (5,6 l/ha).

Bei diesen Beispielen wurde nach der Methode von S.R. Colby (Weeds 15, 20) derjenige Wert E errechnet, der bei einer nur additiven Wirkung der Einzelwirkstoffe zu erwarten war.

Der Berechnung lag folgende Formel zugrunde:

$$E = x + y - \frac{xy}{100}$$

Die Variablen der Gleichung haben folgende Bedeutung:
X Prozentuale Wirkung von Wirkstoff A bei einer Konzentration a
y Prozentuale Wirkung von Wirkstoff B bei einer Konzentration b
E Zu erwartende Wirkung (in %) die durch A + B bei den Aufwandmengen

Tabelle I

| Synergistische herbizide Wirkung der erfindungsgemäßen Mischung aus 2-(1-Imidazolyl)pyrimidin (1.001) und dem Herbizid I.005 bei Nachauflaufapplication im Gewächshaus | | | |
|---|---|---|---|
| Herbizid Nr. I.005 kg/ha | Synergist Nr. 1.001 kg/ha | Amaranthus retroflexus | |
| | | Schädigung in % | E |
| 1.25 | - | 78 | - |
| 0.625 | - | 50 | - |
| - | 0.5 | 0 | - |
| - | 0.125 | 0 | - |
| 1.25 | 0.5 | 100 | 78 |
| 1.25 | 0.125 | 98 | 78 |
| 0.625 | 0.5 | 95 | 50 |
| 0.625 | 0.125 | 80 | 50 |

Tabelle II

| Synergistische herbizide Wirkung der erfindungsgemäßen Mischung aus 2-(1-Imadazolyl)pyrimidin (1.001) und dem Herbizid I.005 bei Nachauflaufapplication im Gewächshaus* | | | |
|---|---|---|---|
| Herbizid Nr. I.005 kg/ha | Synergist Nr. 1.001 kg/ha | Amaranthus retro flexus | |
| | | Schädigung in % | E |
| 0.625 | - | 45 | - |
| - | 0.25 | 0 | - |
| 0.625 | 0.25 | 98 | 45 |

* Alle Formulierungen enthielten zusätzlich 11,2 1/ha einer Ammoniumnitrat-Harnstofflösung (28 % Stickstoff) als Additiv.

Tabelle III

| Synergistische herbizide Wirkung der erfindungsgemäßen Mischung aus 2-(1-Imidazolyl)pyrimidin (1.001) und dem Herbizid I.005 bei Nachauflaufapplication im Gewächschaus | | | |
|---|---|---|---|
| Herbizid Nr. I.005 kg/ha | Synergist Nr. 1001 kg/ha | Amaranthus retroflexus | |
| | | Schädigung in % | E |
| 0.25 | - | 70 | - |
| 0.125 | - | 25 | - |
| - | 0.5 | 0 | - |
| 0.25 | 0.5 | 90 | 70 |
| 0.125 | 0.5 | 70 | 25 |

## Ansprüche

1. Herbizide Mittel, enthaltend mindestens einen herbiziden Wirkstof aus der Gruppe der Benzothiadiazine der Formel I,

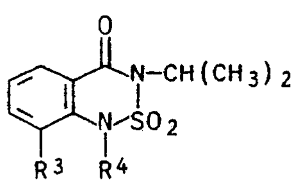

I

in der die Substituenten folgende Bedeutung haben:
$R^3$ Wasserstoff, Halogen, $C_1$-$C_4$-Alkyl oder $C_1$-$C_4$-Alkoxy und
$R^4$ Wasserstoff oder Cyano
oder dessen umweltverträgliches Salz sowie mindestens ein Azolylpyrimidin der Formel IIa oder ein Azolyltriazin der Formel IIb

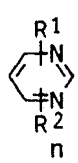

IIa

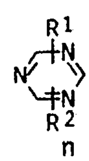

IIb

in denen der Index und die Substituenten folgende Bedeutung haben:
$R^1$ ein fünfgliedriger über sein N-Atom gebundener Heteroaromat, enthaltend zwei oder drei Stickstoffatome, welcher eine Nitrogruppe und/oder ein bis drei der folgenden Reste tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Alkoxycarbonyl, Aminocarbonyl, $C_1$-$C_4$-Alkylaminocarbonyl und/oder Di-$C_1$-$C_4$-Alkylaminocarbonyl;
$R^2$ Halogen; $C_1$-$C_4$-Alkyl; $C_1$-$C_4$-Halogenalkyl; Hydroxy; $C_1$-$C_4$-Alkoxy; $C_1$-$C_4$-Halogenalkoxy; $C_1$-$C_4$-Alkylthio; $C_1$-$C_4$-Halogenalkylthio; Cyano; Nitro; Amino; $C_1$-$C_4$-Alkylamino; $C_3$-$C_7$-Cycloalkylamino; Di-$C_1$-$C_4$-Alkylamino;
Phenyl, Phenoxy, Phenylamino, Benzyl, Benyloxy oder Benzylamino, wobei die aromatischen Reste

ihrerseits ein bis fünf Halogenatome und/oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder ein Rest $R^1$ und n 0, 1 oder 2, wobei die Reste $R^2$ im Falle von n = 2 auch verschieden sein können,
oder deren umweltverträgliche Salze sowie hierfür übliche Zusatzstoffe.

2. Herbizide Mittel nach Anspruch 1, enthaltend als herbiziden Wirkstoff I 3-Isopropyl-1H-2,1,3-benzothiadiazin-4(3H)-2,2-dioxid oder dessen umweltverträgliche Salze.

3. Herbizide Mittel nach Anspruch 1, enthaltend ein Azolylderivat IIa oder IIb, sowie ein Benzothiadiazinderivat I, wobei das Anteilsverhältnis 0,1 : 1 bis 10 : 1 Gew.-Teile beträgt.

4. Herbizide Mittel nach Anspruch 2, enthaltend ein Azolylderivat IIa oder IIb sowie 3-Isopropyl-1H-2,1,3-benzothiadiazin-4(3H)-2,2-dioxid oder dessen umweltverträgliches Salz, wobei das Anteilsverhältnis 0,1 : 1 bis 10 : 1 Gew.-Teile beträgt.

5. 2-(1-Imidazolyl)pyridimin II

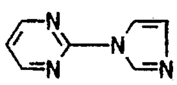

II

und dessen umweltverträgliche Salze.

6. Verfahren zur Bekämpfung unerwünschten Pflanzenwuchses, dadurch gekennzeichnet, daß man ein Azolylpyrimidin- oder -triazin IIa oder IIb gemäß Anspruch 1 und ein Benzothiadiazinderivat I während oder nach dem Auflaufen der unerwünschten Pflanzen vor, bei oder nach der Aussaat der Kulturpflanzen, vor oder während des Auflaufens der Kulturpflanzen gleichzeitig oder nacheinander aufbringt.

7. Verfahren zur Bekämpfung unerwünschten Pflanzenwuches mit Azolylpyrimidinen und -triazinen der Formel IIa und IIb gemäß Anspruch 1 und Benzothiadiaziderivaten der Formel I gemäß Anspruch 1, dadurch gekennzeichnet, daß man die unerwünschten Pflanzen und/oder ihren Lebensraum vor, bei oder nach der Aussat der Kulturpflanzen, vor oder während des Auflaufens der Kulturpflanzen gleichzeitig oder nacheinander mit einer synergistisch wirksamen Menge eines Azolylpyrimidinderivat IIa und/oder IIb und mit einer herbizid wirksamen Menge eines Benzothiadiazinderivates I behandelt.

8. Verfahren nach Anspruch 7, dadurch gekennzeichnet, daß man die Blätter der unerwünschten Pflanzen gleichzeitig oder nacheinander mit einer synergistisch wirksamen Menge eines Azolylpyrimidinderivats IIa und/oder IIb und mit einer herbizid wirksamen Menge eines Benzothiadiazinderivates I behandelt.

9. Verfahren nach den Ansprüchen 6 bis 8, dadurch gekennzeichnet, daß die Kulturpflanzen Gerste, Kultursorghum, Mais, Reis, Weizen, Erbsen, Erdnüsse, Soja und Kulturrasen sind.

Europäisches Patentamt

# EUROPÄISCHER RECHERCHENBERICHT

Nummer der Anmeldung

EP 90 11 8529

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | C.R. WORTHING: "The Pesticide Manual", Auflage 8, 1987, Seiten 63-64, British Crop Protection Council, Thornton Heath, GB<br>--- | 1-9 | A 01 N 43/88<br>A 01 N 43/66<br>A 01 N 43/54<br>C 07 D 403/04 //<br>(A 01 N 43/88<br>A 01 N 43:66<br>A 01 N 43:54 ) |
| A | EP-A-0 257 850 (NISSIN SHOKUHIN K.K.)<br>* Beispiele 4,7,10; Tabelle 1; Patentansprüche *<br>--- | 5 | |
| A | DE-A-1 964 282 (MERCK & CO. INC.)<br>* Insgesamt *<br>----- | 5 | |
| | | | **RECHERCHIERTE SACHGEBIETE (Int. Cl.5)** |
| | | | A 01 N<br>C 07 D |

Der vorliegende Recherchenbericht wurde für alle Patentansprüche erstellt

| Recherchenort | Abschlußdatum der Recherche | Prüfer |
|---|---|---|
| DEN HAAG | 16-01-1991 | DONOVAN T.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument